Europäisches Patentamt

European Patent Office

Office européen des brevets

(11) Veröffentlichungsnummer: **0 255 141 B1**

(12) **EUROPÄISCHE PATENTSCHRIFT**

(45) Veröffentlichungstag der Patentschrift: **01.07.92**

(51) Int. Cl.⁵: **A61K 31/62**

(21) Anmeldenummer: **87111065.6**

(22) Anmeldetag: **30.07.87**

(54) **Wirkstoffkombination enthaltend Diltiazem und Acetylsalicylsäure, deren Herstellung sowie deren Verwendung zur Herstellung von Arzneimitteln mit einer die Thrombozyten-aggregation hemmenden Wirkung.**

(30) Priorität: **31.07.86 DE 3626097**

(43) Veröffentlichungstag der Anmeldung:
**03.02.88 Patentblatt 88/05**

(45) Bekanntmachung des Hinweises auf die
Patenterteilung:
**01.07.92 Patentblatt 92/27**

(84) Benannte Vertragsstaaten:
**AT BE CH DE ES FR GB GR IT LI LU NL SE**

(56) Entgegenhaltungen:
**EP-A- 0 214 881**

**BIOLOGICAL ABSTRACTS/RRM 28064647;
K.L. CHAN et al.: "Coronary thrombosis and
subsequent lysis after a marathon", & JOUR-
NAL OF THE AMERICAN COLLEGE OF CAR-
DIOLOGY(US) 1984. vol. 4, no. 6, p.1322-1325**

**CHEMICAL ABSTRACTS, Band 106, Nr. 3, 19.
Januar 1987 1987, Seite 53, Zusammenfassung Nr. 12658r, Columbus, Ohio, US; M.E.
RING et al.: "Effects of oral diltiazem on
platelet function: alone and in combination
with "low dose" aspirin", & THROMB. RES.**

**1986, 44(3), 391-400**

(73) Patentinhaber: **GÖDECKE AKTIENGESELL-
SCHAFT
Salzufer 16
W-1000 Berlin 10(DE)**

(72) Erfinder: **Satzinger, Gerhard, Dr.
Im Mattenbühl 7
W-7809 Denzlingen(DE)**
Erfinder: **Wolf, Günter, Dr.
Wildtalstrasse 40
W-7800 Freiburg(DE)**
Erfinder: **Osswald, Hartmut, Prof. Dr.
Kiefernweg 1
W-7808 Waldkirch 2(DE)**
Erfinder: **Weiershausen, Ute
Bundesstrasse 70
W-7803 Gundelfingen(DE)**

**Beschreibung**

Eine rationelle Prophylaxe und Therapie muß sich an der Pathogenese der jeweiligen Erkrankung orientieren. Bezüglich der Gefäßverschlußkrankheiten steht den Thrombozyten eine maßgebliche Bedeutung zu. Dies gilt für die initiale Thrombogenese im arteriellen System so gut wie für die Entstehung von Gerinnungsthromben in der venösen Zirkulation. Experimentelle Erkenntnisse neuerer Art weisen ferner darauf hin, daß Arteriopathien im weitesten Sinn als das Resultat einer gestörten Beziehung zwischen Gefäßwand (dem Endothel) und den Thrombozyten zu sehen sind. Dieser Sachverhalt liefert neue Ansätze für entsprechende kausale therapeutische Maßnahmen.

Da die in vivo Thrombozytenaggregation mit hoher Wahrscheinlichkeit als Folge der gleichzeitigen Aktivierung oder Ausschüttung mehrerer endogener Faktoren [wie der Platelet Activating Factor (PAF), Adrenalin, ADP/ATP, Lipoxigenase- und Cyclooxigenase-Produkte der Arachidonsäure] erfolgt, kann von der Wirkung eines Cyclooxigenase-Hemmers (wie Acetylsalicylsäure (ASS), Sulfinpyrazon) allein kein umfassender therapeutischer Effekt erwartet werden.

Die EP-A- 0 214 881 beschreibt eine mögliche Kombination von Acetylsalicylsäure mit Diltiazem.

Das Journal of the American College of Cardiology Vol. 4 Nr.6 (1984) S. 1322-1325 beschreibt zwar, daß einem Patienten nach einem Infarkt Diltiazem und Acetylsalicylsäure gegeben wurde, jedoch wird weder über die verabreichte Dosis noch über einen synergistischen Effekt der Wirkstoffkombination in einer Dosiseinheit etwas ausgesagt.

Die vorliegende Erfindung betrifft eine neuartige Zubereitung zur Therapie und Prophylaxe thromboembolischer Erkrankungen wobei als eine Wirkstoffkombination Diltiazem und Acetylsalizylsäure (ASA) in einer Wirkstoffkombination eingesetzt wird, sowie deren Verwendung.

Unter thromboembolischen Erkrankungen werden hierbei verstanden:
- Periphere arterielle und venöse Verschlußkrankheiten/Arteriorpathien (Verhütung thromboembolischer Komplikationen)
- Koronare Herzkrankheit (Prophylaxe nach Herzinfarkt; Prävention von Venethrombosen und arteriellen Embolien)
- Cerebrovaskuläre Gefäßerkrankungen (Ischämien; Insult als Folge thromboembolischer Inzidenzen)
- Arteriosklerose (Reduktion von Risikofaktoren)
- Migräne (Prophylaxe und Therapie)
- Raynaud-Syndrom (Prophylaxe und Therapie bei thromboembolischer Disposition)

Die antithrombotische Wirkung der ASS ist bekannt (Med. Klin 76 (1981), S. 692). Die optimale Dosis ist nach wie vor umstritten. Die Empfehlungen schwanken zwischen 300 mg/Tag und 3 x 500 mg/Tag bzw. 4 x 325 mg/Tag. ASS bewirkt eine irreversible Hemmung der Cyclooxigenase, eines Enzyms, welches in den Thrombozyten $TXA_2$ produziert; ohne $TXA_2$ findet keine Blutstillung (als erwünschter Effekt), und keine, für die Thrombogenese erforderliche Aggregation der Blutplättchen statt. Gleichzeitig hemmt ASS jedoch auch die Cyclooxigenase der Intima der Gefäße, so daß der endogene Aggregationshemmer $PGI_2$ nicht gebildet werden kann; dadurch erhält ASS eine thrombogene Potenz. Da die Cyclooxigenase der Thrombozyten empfindlicher gegenüber ASS ist und langsamer regeneriert wird, als die der inneren Gefäßwandschicht, vermutet man, daß über die Dosis eine gewisse Differenzierung der ASS-Effekte erreicht werden kann.

Zum einen wegen der Reduktion der ASS-Nebenwirkungen, die beträchtlich sein können und sich vor allem im Gastrointestinaltrakt und in der Niere manifestieren, und zum anderen aus den obengenannten Gründen ist es von entscheidender therapeutischer Bedeutung,die ASS-Dosis so niedrig wie möglich zu halten. Dies gelingt für den erfindungsgemäßen Zweck durch eine Kombination von ASS mit Diltiazem.

Es ist überraschend, daß Diltiazem, ein bekannter Koronarvasodilator, bereits in Kombination mit außerordentlich niedrigen Dosen ASS, dessen therapeutische, thrombozyten-aggregationshemmende Wirkung in synergistischer Weise verstärkt. Eine schwache eingene, die Aggregation der Blutplättchen hemmende Wirkung des Diltiazem wäre aufgrund der Calcium-antagonistischen Wirkung deutbar, da die Aktivierung der Phospholipase $A_2$ der Thrombozyten, die Aktivierung des Actomyosins der Plättchen, möglicherweise auch die des Platelet Derived Growth Faktors (PDG) der Intima unter der Kontrolle von $Ca^{2+}$-Ionen stehen. Es war aber in keiner Weise vorherzusehen, daß die Kombination von subtherapeutischen Dosen von Diltiazem und ASS einen starken antiaggregatorischen Effekt ausüben würde. Die erfindungsgemäße Kombination stellt somit wegen der erhöhten Sicherheit und Wirksamkeit einen echten therapeutischen Fortschritt dar.

Eine enterale Dosiseinheit besteht aus >60 mg bis 180 mg Diltiazem und 10 - 300 mg Acetylsalicylsäure. Ganz besonders bevorzugt ist eine enterale Dosiseinheit von 80 bis 100 mg Diltiazem und 50 bis 100 mg Acetylsalicylsäure.

Als Salze kommen übliche Säureadditionssalze des Diltiazems mit organischen oder anorganischen Säuren infrage. Die Salze werden in üblicher Weise durch Neutralisation der Base mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpelsäure, Salicylsäure, Ascorbinsäure, Malonsäure oder Bernsteinsäure in Frage.

Wegen der basischen Natur des Diltiazem kann ASS vorteilhaft unmittelbar als Salzbildner wirken, insbesondere weil dieses Salz die Wirkstoffe in einer sehr guten Relation enthält. 130 mg des neuen Diltiazem-acetylsalicylats enthalten dann ca. 90 mg Diltiazem-Wirkstoff und 40 mg ASS-Wirkstoff.

Eine Zubereitung mit 100 mg Diltiazem-acetylsalicylat pro enterale Dosierungseinheit enthält damit 30,3 mg Acetylsalicylsäure und 69,7 mg Diltiazem. Das entspricht einem Gew.Verhältnis von 1 : 2,3.

Diltiazem, oder ein Säureadditionssalz davon, und Acetylsalicylsäure, sowie Diltiazem-Acetylsalicylat können in üblichen Zubereitungen und in Mischungen mit üblichen pharmazeutisch annehmbaren Trägern oder Verdünnungsmitteln angewendet werden.

Die erfindungsgemäßen Zubereitungen können in flüssiger oder fester Form oral oder parenteral appliziert werden. Als Injektionslösung kommt vor allem Wasser zur Anwendung, welche die bei Injektionslösungen üblichen Zusätze wie Stabilisierungsmittel, Lösungsvermittler oder Puffer enthält.

Die Zubereitungen können als übliche galenische Formulierungen, wie Tabletten oder Kapseln vorliegen. Sie werden hergestellt, indem man Diltiazem oder ein pharmakologisch akzeptables Säureadditionssalz in an sich bekannter Weise zusammen mit Acetylsalicylsäure in einen pharmakologisch unbedenklichen Trägerstoff und gegebenenfalls geeigneten Zusätzen einarbeitet.

Derartige Zusätze sind z.B. Tartrat- und Citrat-Puffer, Ethanol, Komplexbildner (wie Äthylendiamintetraessigsäure und deren nichttoxische Salze) sowie hochmolekulare Polymere (wie flüssiges Polyäthylenoxid) zur Viskositätsregulierung, Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Poläthylenglykol); für orale Applikation geeignete Zubereitungen können gewünschtenfalls zusätzliche Geschmacks- und/oder Süßstoffe enthalten.

Die enteral verabreichten Einzeldosen der neuen Zubereitung liegen im Bereich von 50 bis 400 mg.

Wegen der bekannten Stabilitätsprobleme von ASS-Lösungen ist bei Injektionsformen vor allem an Lyophilisate gedacht, die erst kurz vor der Anwendung in Lösung gebracht werden.

Lyophilisate werden natürlich entsprechend niedriger dosiert, nämlich im Bereich von 5 - 100 mg pro Einzeldosis im selben Gewichtsverhältnis wie die enteralen Kombinationen von Diltiazem und ASS.

Die Erfindung wird in dem nachfolgenden Vergleichsversuch näher erläutert:

VERGLEICHSVERSUCH

Kollageninduzierte Thrombozytenaggregation

Material und Methoden

Als Versuchstiere dienten männliche Sprague Dawley SIV 50-Ratten mit einem Körpergewicht von 200 g. Die in Wasser gelösten Testsubstanzen wurden zweimalig den Tieren per Schlundsonde verabreicht: Die erste Gabe erfolgte un 15.00 Uhr des Vortages, die zweite Gabe um 08.00 Uhr des Versuchstages. Nach der ersten Gabe wurden den Ratten das Futter entzogen, sie erhielten aber weiterhin Wasser. Die Tiere erhielten pro Gabe in einem Volumen von 1 ml/200 g Körpergewicht folgende Dosierungen:

| | |
|---|---|
| Placebo: | nur Wasser |
| Dosierung 1: | 30 mg Diltiazem/kg |
| Dosierung 2: | 100 mg Diltiazem/kg |
| Dosierung 3: | 5 mg Acetylsalicylsäure/kg |
| Dosierung 4: | 30 mg Diltiazem/kg und |
| | 5 mg Acetylsalicylsäure/kg |

Um 09.00 Uhr des Versuchstages, also 1 Stunde nach der zweiten Gabe wurde den Tieren unter Äthernarkose Blut aus dem retroorbitalen Venenplexus entnommen. Neun Teile Blut wurden mit einem Teil 3,8 % (w/v) Tri-Na-Citrat-Lösung versetzt. Nach niedrigtouriger Zentrifugation des Gemisches bei Raumtemperatur wurde das Plättchen-reiche Plasma (PRP, entsprechend dem Erythrozyten-freien Oberstand) abgenommen und auf eine Standard-Konzentration von 400.000/$\mu$] eingestellt. Der Aggregationstest wurde nach der Methode von BORN (Nature 194,S. 927-929 1962) durchgeführt. Als Meßinstrument diente ein Universalaggregometer (Braun, Melsungen) verbunden mit einem Eppendorf-Photometer 1100 M (Netheler und Hinz, Hamburg).

Im Versuch wurden jeweils 800 $\mu$l PRP im Aggregometer 3 min. bei 37°C äquilibriert und sodann durch Zugabe von 35 $\mu$l Kollagensuspension (Kollagenreagenz HORM®, Hormon Chemie, München) die Aggregation ausgelöst. Ein Kompensationsschreiber registrierte in dem folgenden 5 min. die Änderung der Transmission. Mit zunehmender Aggregation nimmt in diesem Test die Transmission zu, die Extinktion ab. Die 5 min. nach Kollagenzugabe registrierten Änderungen wurden ausgewertet, sie sind in der Tabelle aufgeführt. Die Werte sind in % angegeben, wobei die Extinktionsdifferenzen zwischen PRP und Plättchenfreiem Plasma als 100%-Wert dienten. Die erhaltenen Aggregationswerte sind der nachfolgenden Tabelle I zu entnehmen.

T A B E L L E   I

|  |  | Aggregation $\bar{x} \pm$ S.D. |
|---|---|---|
| Dosierung | Placebo | 65 $\pm$ 12 |
| 1 | 30 mg/kg D. | 63 $\pm$ 10 |
| 2 | 100 mg/kg D. | 58 $\pm$ 10 |
| 3 | 5 mg/kg ASS | 37 $\pm$ 12 |
| 4 | 30 mg/kg D.<br>+ 5 mg/kg ASS | 19 $\pm$ 15 |

( $\bar{x} \pm$ S.D. aus jeweils 6 Einzelversuchen)

Aus der Tabelle I ist ersichtlich, daß Dosierung 4 zu einem überraschend niedrigen Aggregationswert führt.

Beispiel

Diltiazem - acetylsalicylat

731,4 mg (1,76 m mol) Diltiazem werden zusammen mit 317,9 mg (1,76 m mol) Acetylsalicylsäure in 10 ml Wasser gelöst und die Lösung im Vakuum bei Raumtemperatur zur Trockne gebracht. Der amorphe, farblose Rückstand wird zerkleinert. Ausbeute ca. 1g weißes Pulver. Für die spektroskopischen NMR-Untersuchungen wird die Substanz in $CDCl_3$ gelöst.

Das Salz weist im NMR-Spektrum die zu erwartende chemische Verschiebung auf, wie sie in der folgenden Tabelle II dokumentiert ist:

T A B E L L E   II

Zuordnung und Gegenüberstellung der NMR-Signale
des Diltiazem - acetylsalicylat - Salzes.

| Fragment | Chem. Verschiebung $\delta$ in ppm | | $\|\Delta\delta\|$ | |
| --- | --- | --- | --- | --- |
| | BASE | SALZ | | |
| $\overset{\overset{O}{\|\|}}{C\text{-}CH_3}$ | 1,90 | 1,90 | – | S |
| $N(CH_3)_2$ | 2,27 | 2,60 | 0,33 | S |
| $N\text{-}CH_2$ | 2,46 | 2,94 | 0,48 | M |
| | 2,69 | 3,15 | 0,46 | M |
| $O\text{-}CH_3$ | 3,82 | 3,82 | – | S |
| $N\text{-}CH_2$ | 3,71 | 4,08 | 0,37 | M |
| | 4,42 | 4,46 | 0,04 | M |
| C-H | 5,01 | 5,01 | – | D |
| C-H | 5,16 | 5,13 | 0,03 | D |
| p-Methoxy-aromat m. =CH- | 6,89 | 6,90 | 0,01 | D |
| o. =CH- | 7,44 | 7.39 | 0,05 | D |
| aromat. H Grundkörper | 7,26 | 7,25 | 0,01 | M |
| | 7,48 | 7,5 | , | M |
| | 7,48 | 7,5 | , | M |
| | 7,69 | 7,68 | 0,01 | DM |
| Acetyl-salicylsre. $\overset{\overset{O}{\|\|}}{C\text{-}CH_3}$ | | 2,25 | | S |
| arom. =CH- | | 7,04 | | DD |
| | | 7,25 | | M |
| | | 7,5 | | M |
| | | 7,96 | | DD |

Patentansprüche
Patentansprüche für folgende Vertragsstaaten : BE, CH, DE, FR, GB, IT, LI LU, NL, SE

1. Wirkstoffkombination zur Verstärkung der antithrombotischen Wirkung von Acetylsalicylsäure dadurch gekennzeichnet, daß diese Acetylsalicylsäure in einer Menge von 10 - 300 mg und Diltiazem oder ein pharmazeutisch akzeptables Salz desselben in einer Menge >60 - 180 mg (bezogen auf Wirkstoffbase) in einer Dosiseinheit enthält.

2. Wirkstoffkombination nach Anspruch 1 enthaltend Diltiazemacetylsalicylat.

3. Wirkstoffkombination nach Anspruch 1 oder 2 in Form eines Lyophilisats.

4. Diltiazem-acetylsalicylat

5. Verwendung von Diltiazem und Acetylsalicylsäure als Wirkstoffkombination nach Anspruch 1 bis 3 zur Herstellung eines Arzneimittels für die Behandlung thromboembolischer Erkrankungen.

6. Verfahren zur Herstellung einer Wirkstoffkombination zur Verstärkung der antithrombotischen Wirkung von Acetylsalicylsäure nach Anspruch 1 bis 3, dadurch gekennzeichnet, daß man das Diltiazem oder ein pharmakologisch akzeptables Säureadditionsalz desselben zusammen mit der Acetylsalicylsäure in einen pharmakologisch unbedenklichen Trägerstoff einarbeitet.

7. Verfahren zur Herstellung von Diltiazem-acetylsalicylat, dadurch gekennzeichnet, daß man Diltiazem in Lösung in einmolarem Verhältnis mit Acetylsalicylsäure umsetzt.

8. Verfahren nach Anspruch 6 bis 7, dadurch gekennzeichnet, daß man einen Gefriertrocknungsschritt anschließt.

**Patentansprüche für folgende Vertragsstaaten : AT, ES, GR**

1. Wirkstoffkombination zur Verstärkung der antithrombotischen Wirkung von Acetylsalicylsäure dadurch gekennzeichnet, daß sie Diltiazem oder ein pharmazeutisch akzeptables Säureadditionsalz desselben in Kombination mit Acetylsalicylsäure enthält.

2. Wirkstoffkombination nach Anspruch 1, dadurch gekennzeichnet, daß Acetylsalicylsaure in einer Menge von 10 - 300 mg und Diltiazem oder ein Salz desselben in einer Menge von >60 - 180 mg (bezogen auf Wirkstoffbase) in einer Dosiseinheit vorliegen.

3. Wirkstoffkombination nach Anspruch 1 oder 2 enthaltend Diltiazem-acetylsalicylat.

4. Wirkstoffkombination nach Anspruch 1 bis 3 in Form eines Lyophilisats.

5. Diltiazem-acetylsalicylat

6. Verwendung von Diltiazem und Acetylsalicylsäure als Wirkstoffkombination nach Anspruch 1 bis 3 zur Herstellung eines Arzneimittels für die Behandlung thromboembolischer Erkrankungen.

7. Verfahren zur Herstellung eines Arzneimittels zur Verstärkung der antithrombotischen Wirkung von Acetylsalicylsäure, dadurch gekennzeichnet, daß man Diltiazem oder ein pharmakologisch akzeptables Säureadditionsalz desselben zusammen mit Acetylsalicylsäure in einen pharmakologisch unbedenklichen Trägerstoff einarbeitet.

8. Verfahren nach Anspruch 7, dadurch gekennzeichnet, daß Acetylsalicylsaure in einer Menge von 10 - 300 mg zusammen mit Diltiazem oder einem Salz desselben in einer Menge von >60 - 180 mg (bezogen auf Wirkstoffbase pro Dosiseinheit) eingearbeitet wird.

9. Verfahren zur Herstellung von Diltiazem-acetylsalicylat, dadurch gekennzeichnet, daß man Diltiazem in Lösung in einmolarem Verhältnis mit Acetylsalicylsäure umsetzt.

10. Verfahren nach Anspruch 7 bis 9, dadurch gekennzeichnet, daß man einen Gefriertrocknungsschritt anschließt.

**Claims**

**Claims for the following Contracting States : BE, CH, DE, FR, GB, IT, LI, LU, NL, SE**

1. Active material combination for the strengthening of the antithrombotic action of acetylsalicylic acid, characterised in that it contains acetylsalicylic acid in a quantity of 10 - 300 mg and diltiazem or a pharmaceutically acceptable salt thereof in a quantity of >60 - 180 mg (relative to active material base) in a dosage unit.

2. Active material combination according to claim 1 containing diltiazem acetylsalicylate.

3. Active material combination according to claim 1 or 2 in the form of a lyophilisate.

4. Diltiazem acetylsalicylate.

5. Use of diltiazem and acetylsalicylic acid as an active material combination according to claim 1 to 3 for the preparation of a medicament for the treatment of thromboembolic diseases.

6. Method for the preparation of an active material combination for the strengthening of the antithrombotic action of acetylsalicylic acid according to claim 1 to 3, characterised in that the diltiazem or a pharmacologically acceptable acid-addition salt thereof is incorporated together with the acetylsalicylic acid into a pharmacologically safe carrier material.

7. Method for the preparation of diltiazem acetylsalicylate, characterised in that diltiazem is reacted in solution in unimolar ratio with acetylsalicylic acid.

8. Method according to claim 6 to 7, characterised in that a freeze-drying step follows.

**Claims for the following Contracting States : AT, ES, GR**

1. Active material combination for the strengthening of the antithrombotic action of acetylsalicylic acid, characterised in that it contains diltiazem or a pharmaceutically acceptable acid-addition salt thereof in combination with acetylsalicylic acid.

2. Active material combination according to claim 1, characterised in that acetylsalicylic acid in a quantity of 10 - 300 mg and diltiazem or a salt thereof in a quantity of >60 - 180 mg (relative to active material base) are present in a dosage unit.

3. Active material combination according to claim 1 or 2 containing diltiazem acetylsalicylate.

4. Active material combination according to claim 1 to 3 in the form of a lyophilisate.

5. Diltiazem acetylsalicylate.

6. Use of diltiazem and acetylsalicylic acid as an active material combination according to claim 1 to 3 for the preparation of a medicament for the treatment of thromboembolic diseases.

7. Method for the preparation of a medicament for the strengthening of the antithrombotic action of acetylsalicylic acid, characterised in that diltiazem or a pharmacologically acceptable acid-addition salt thereof is incorporated together with acetylsalicylic acid into a pharmacologically safe carrier material.

8. Method according to claim 7, characterised in that acetylsalicylic acid in a quantity of 10 - 300 mg is incorporated together with diltiazem or a salt thereof in a quantity of >60 - 180 mg (relative to active material base per dosage unit).

9. Method for the preparation of diltiazem acetylsalicylate, characterised in that diltiazem is reacted in solution in unimolar ratio with acetylsalicylic acid.

10. Method according to claim 7 to 9, characterised in that a freeze-drying step follows.

**Revendications**
**Revendications pour les Etats contractants suivants : BE, CH, DE, FR, GB, IT, LI, LU, NL, SE**

1.  Association de substances actives pour le renforcement de l'effet antithrombotique de l'acide acétylsalicylique, caractérisée en ce que cette association contient dans une dose unitaire de l'acide acétylsalicylique en une quantité de 10 à 300 mg et du diltiazème ou un de ses sels pharmaceutiquement acceptables en une quantité >60 à 180 mg (par rapport à la base de la substance active).

2.  Association de substances actives selon la revendication 1, contenant de l'acétylsalicylate de diltiazème.

3.  Association de substances actives selon la revendication 1 ou 2, sous la forme d'un lyophilisat.

4.  Acétylsalicylate de diltiazème.

5.  Utilisation de diltiazème et d'acide acétylsalicylique comme association de substances efficaces selon les revendications 1 à 3 pour la préparation d'un médicament destiné au traitement de maladies thromboemboliques.

6.  Procédé de préparation d'une association de substances efficaces pour le renforcement de l'effet antithrombotique de l'acide acétylsalicylique selon les revendications 1 à 3, caractérisé en ce que l'on incorpore du diltiazème ou l'un de ses sels d'addition d'acide pharmaceutiquement acceptables en même temps que de l'acide acétylsalicylique sur un support pharmaceutiquement neutre.

7.  Procédé de préparation d'acétylsalicylate de diltiazème, caractérisé en ce que l'on fait réagir du diltiazème, en solution selon un rapport monomolaire, avec de l'acide acétylsalicylique.

8.  Procédé selon les revendications 6 à 7, caractérisé en ce que l'on procède ensuite à une étape de séchage par lyophilisation.

**Revendications pour les Etats contractants suivants : AT, ES, GR**

1.  Association de substances actives pour le renforcement de l'effet antithrombotique de l'acide acétylsalicylique, caractérisée en ce qu'elle contient du diltiazème ou l'un de ses sels d'addition d'acide pharmaceutiquement acceptables en association avec l'acide acétylsalicylique.

2.  Association de substances actives selon la revendication 1, caractérisée en ce que l'acétylsalicylique est présent en une quantité de 10 à 300 mg et le diltiazème ou l'un de ses sels en une quantité de >60 à 180 mg (par rapport à la base de substances actives) dans une dose unitaire.

3.  Association de substances actives selon la revendication 1 ou 2, contenant de l'acétylsalicylate de diltiazème.

4.  Association de substances actives selon les revendications 1 à 3, sous la forme d'un lyophilisat.

5.  Acétylsalicylate de diltiazème.

6.  Utilisation de diltiazème et d'acide acétylsalicylique comme substances actives selon les revendications 1 à 3 pour la préparation d'un médicament destiné au traitement de maladies thromboemboliques.

7.  Procédé de préparation d'un médicament pour le renforcement de l'effet antithrombotique de l'acide acétylsalicylique, caractérisé en ce que l'on incorpore du diltiazème ou un de ses sels d'addition d'acide pharmaceutiquement acceptables en même temps que de l'acide acétylsalicylique sur un support pharmaceutiquement neutre.

8.  Procédé selon la revendication 7, caractérisé en ce que l'acide acétylsalicylique est incorporé en quantité de 10 à 300 mg en même temps que du diltiazème ou l'un de ses sels en quantité de >60 à 180 mg par rapport à la substance de base par dose unitaire).

9. Procédé de préparation d'acétylsalicylate de diltiazème, caractérisé en ce que l'on fait réagir du diltiazème, en solution selon un rapport monomolaire, avec l'acide acétylsalicylique.

10. Procédé selon les revendications 7 à 9, caractérisé en ce que l'on procède ensuite à une étape de lyophilisation.